# EUROPEAN PATENT SPECIFICATION

(11) **EP 3 848 349 B1**
(45) Date of publication and mention of the grant of the patent: **15.03.2023**
(21) Application number: 20461587.6
(22) Date of filing: 01.12.2020
(51) Int. Cl.: C07C 227/18, C07C 229/16, C07C 229/76

(54) **A PROCESS FOR THE PREPARATION OF SALTS OF N,N'-DISUBSTITUTED ETHYLENEDIAMINE-N,N'-DIACETIC ACID DERIVATIVES AND THEIR USE**
EIN VERFAHREN ZUR HERSTELLUNG VON SALZEN N,N'-DISUBSTITUIERTEN ETHYLENDIAMIN-N,N'-DIESSIGSÄUREDERIVATEN UND IHRE VERWENDUNG
UN PROCÉDÉ DE PRÉPARATION DES SELS DES DÉRIVÉS D'ACIDE ÉTHYLÈNEDIAMINE-N,N'-DIACÉTIQUE N,N'-DISUBSTITUÉS ET LEUR UTILISATION

(43) Date of publication of application: 14.07.2021
(73) Proprietor: Przedsiebiorstwo Produkcyjno-Consultingowe ADOB Sp. z o.o., 61-070 Poznan (PL)
(72) Inventor: Nawrocki, Adam, 61-306 Poznan (PL); Olszewski, Radoslaw, 61-038 Poznan (PL); Ciarka, Kamil Krzysztof, 61-619 Poznan (PL); Michalek, Bernard Tomasz, 61-655 Poznan (PL); Stegient-Nowicka, Joanna Ewa, 61-160 Czapury (PL); Matyniak, Magdalena, 60-682 Poznan (PL); Wilk, Tomasz, 62-028 Kozieglowy (PL); Pernak, Juliusz, 60-573 Poznan (PL)
(74) Representative: Sitkowska, Jadwiga

(56) References cited:
- EP-B1- 2 039 679
- WO-A1-2016/207266
- US-A- 2 967 196

## Description

### Field of the invention

The invention relates to a process for the preparation of salts of *N,N*'-disubstituted ethylenediamine-*N,N*'-diacetic acid derivatives. Said derivatives find their use for the preparation of chelates comprising microelements such as Fe or Zn. Chelates are used as fertilizers for reduction of nutrients deficiencies in the cultivations carried out in soils of high pH and high hydrocarbonate contents.

### Background art

Known chelating agents for metals, including especially Fe(III) and Zn(II), used for the preparation of micronutrient fertilizers comprising metal chelates are *N,N*'-disubstituted ethylenediamine-*N*,*N*'-diacetic acid derivatives, namely HBED (*N,N'*-di(2-hydroxybenzyl)-ethylenediamine-*N*,*N*'-diacetic acid) and its derivative 5Me-HBED with methyl substituent in the aromatic ring (*N*,*N*'-di(2-hydroxy-5-methylbenzyl)ethylenediamine-*N*,*N*'-diacetic acid), known also as HJB or 5M-HBED.

Said chelating agents form with Fe(III) and Zn(II) water-soluble chelates having exceptionally high stability in solutions. It is a major advantage when said chelating agents are used for binding metal micronutrients, such as Fe(III) and Zn(II), in the preparation of agricultural fertilizers, especially for areas that are prone to iron and zinc deficiencies. Such chelates are prepared in the reaction of the salt of a metal, for example Fe or Zn, with chelating agent in the form of its sodium or potassium salt, or acid addition salt.

Processes for the preparation of HBED and its derivative 5Me-HBED are known.

US2967196 discloses a process for the preparation of *N*,*N*'-di(2-hydroxybenzyl)-ethylenediamine-*N,N*'-diacetic acid (HBED) derivatives substituted at phenyl ring, including 5Me-HBED, in Mannich reaction. The process comprises ternary reaction between *p*-cresol, formaldehyde and ethylenediaminediacetic acid sodium salt to obtain a desired product. Disadvantage of this method is simultaneous production of *o,o*'-dihydroxydiphenylmethane. Modification of this process is also disclosed, wherein in the first step formaldehyde is reacted with ethylenediaminediacetic acid sodium salt to obtain dimethylol derivative which is condensed in the second step with substituted phenol in the *para* position. US2967196 discloses also preparation of Fe(III) chelates in the reaction of aqueous solution of sodium salt of chelating agent thus obtained with iron(III) chloride.

F. L. Eplattenier et al. in J. Am. Chem. Soc. 1967, 89, 4, 837-843 disclose a process for the preparation of *N,N*'-di(2-hydroxybenzyl)ethylenediamine-*N,N*'-diacetic acid (HBED) comprising reaction of ethylenediamine-*N,N*'-diacetic acid sodium salt with o-acetoxybenzyl bromide. The reaction is carried out in ethyl alcohol in the period of two hours. HBED thus obtained is recovered from the reaction mixture as dihydrochloride by acidification of the reaction system with hydrochloric acid to the pH in the range 1-1.5. The yield of the process is 52%.

In a similar manner HBED dihydrochloride is obtained according to US3632637. To ethylenediamine-*N,N*'-diacetic acid disodium salt dissolved in a 70% ethyl alcohol - 30 % water mixture *o*-acetoxybenzyl chloride is added, and obtained chloride is neutralized with sodium hydroxide for 6 hours. After completion of the reaction, ethyl alcohol is evaporated from the reaction mixture. The mixture is acidified to the pH=1.0 to recover the product as dihydrochloride. The yield of the process is 70%.

US3632637 discloses also a process for the preparation of *N*-(2-hydroxybenzyl)-aminopolycarboxylic acids, including HBED, which comprises reaction of *o*-hydroxybenzyl alcohol with esterifying agent, selected from the group comprising low-molecular weight fatty acids and their anhydrides, then halogenation of ester compound thus obtained with the solution of a halogen hydride, reaction of *o*-acyloxybenzyl halide thus obtained with suitable aminoacetic acid in the presence of an alkaline agent, and removal of phenol-protecting ester group.

US6646157 discloses a process of the preparation of HBED mono- or disodium salt by *N*-alkylation of *N,N*'-di(2-hydroxybenzyl)ethylenediamine with *tert*-butyl ester of 2-halogenoacetic acid in the presence of potassium carbonate and dimethylsulfoxide, followed by hydrolysis with formic acid to obtain HBED in acid form, which is then neutralized with aqueous solution of sodium hydroxide to obtain water soluble form. The use of multistep synthesis, organic solvents and multistage purification process of the product leads to obtaining the product with low yield (26%) and compromises the use of this process in a technical scale.

A. L. Martell et al. in Can. J. Chem. 1986, 64, 449-456 describe a process of the preparation of HBED in the reaction of *N,N*'-di(2-hydroxybenzyl)ethylenediamine with formaldehyde and hydrogen cyanide. *N*,*N*'-Di(2-hydroxybenzyl)ethylenediamine-*N*,*N*'-diacetonitrile thus obtained does not hydrolyze to HBED both in acidic and basic environment; under reaction conditions *N,N*'-di(2-hydroxybenzyl)monoketopiperazine is obtained. *N,N'-*Di(2-hydroxybenzyl)ethylenediamine-*N,N*'-diacetonitrile is converted to *N,N*'-di(2-hydroxybenzyl)ethylenediamine-*N,N*'-diamide in the reaction with hydrogen peroxide at 5-10°C, in the presence of catalytic amounts of sodium hydroxide. HBED diamide thus obtained does not hydrolyze to HBED neither under acidic nor basic conditions, or in the presence of sodium nitrate in acetic acid. Hydrolysis of diamide is possible only with the use of catalytic amounts of copper(II) or iron(III).

N. A. Thiele et al. in Chem. Med. Chem 2016, 11, 1596-1599 describe a process of the preparation of HBED in the form of dimesyl salts in a four-step reaction. Obtained compounds in the form of HBED carboxylic and borate esters are hydrolyzed in the presence of hydrogen peroxide. Obtained compounds find their use in the medicine as an alternative for desferrioxamine in removal of excess of iron from the body by transfusion.

From US303879 there is known a process of the preparation of chelating agents - HBED derivatives containing alkyl substituent/substituents in phenolic group, in a two-stage reaction, which comprises condensation of ethylenediamine-*N,N*'-diacetic acid disodium salt with formaldehyde, followed by condensation of the compound thus obtained with phenol derivatives in aqueous/alcoholic medium at the temperature below 100°C and pH in the range 8-10. According to the described process, chelating agent is obtained with the yield about 70-80%. Obtained chelating agent can bind iron. Preparation of a chelating agent that does not include alkyl substituents in phenolic group was not described.

Modification of the above process is a process of the preparation of HBED according to WO2016/207266, comprising reaction between formaldehyde, ethylenediaminediacetic acid (EDDA) or its salt, and phenol in the presence of 0.2 to 1.1 mol of alkaline metal ions, calculated on introduced EDDA. Characteristic for the process is that not less than 50 mol% of alkaline metal ions are potassium. The process gives HBED with the yield about 80-85%. The process comprises the use of excess of phenol, however the possibility of its return to the production cycle was not described.

A process of the preparation of HBED described in US2001039295 and WO0146114 comprises reaction of salicylaldehyde and ethylenediamine to obtain salen, then reduction of salen to salan using sodium borohydride or hydrogen, alkylation of the obtained compound with haloacetic acid *tert*-butyl ester, and finally hydrolysis of *tert*-butyl ester to HBED. Reactions are carried out in organic solvents such as THF, DMSO, in several steps, and using expensive raw materials, such as salicylaldehyde. This significantly raises the costs of the preparation of the product.

From EP2039679B1 there is known a process for the preparation of HBED and 5Me-HBED in the reaction of reductive amination of salan (*N,N*'-di(2-hydroxybenzyl)-ethylenediamine) with stoichiometric amount of glyoxalic acid. Reductive amination takes place in the presence of a catalyst, especially a palladium or nickel one, in the hydrogen environment at enhanced pressure. Reaction product is recovered from the reaction mixture as monohydrochloride or sodium, ammonium or potassium salts.

### The summary of the invention

The present invention solves the problem of providing a new process of the preparation of the salts of *N*,*N*'-disubstituted derivatives of ethylenediamine-*N,N*'-diacetic acid HBED and 5Me-HBED, in particular disodium and dipotassium salts, and addition salts thereof with hydrochloric and sulfuric acid. The present of the invention utilizes easy accessible and relatively non-toxic starting materials and does not require the use of phenol or its derivatives or formaldehyde.

### Detailed description of the invention

The object of the invention is therefore a process of the preparation of the salts of *N,N'-*disubstituted ethylenediamine-*N,N*'-diacetic acid derivatives, said salts being represented by the formula (IA) 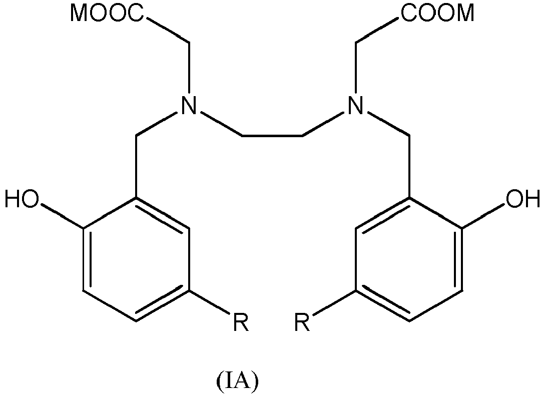
wherein both R have the same meaning and represent hydrogen atom or CH₃ group, and both M have the same meaning and represent sodium or potassium cation,
which comprises alkylation reaction of *N*-substituted glycine derivative of the formula (II) 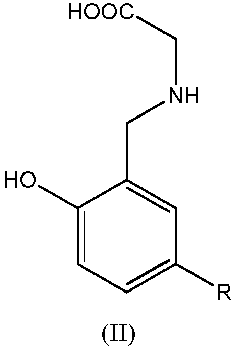
wherein R has the meaning as given for formula (IA),
with 1,2-dichloroethane in water in the presence of MOH hydroxide, wherein M represents sodium or potassium cation, respectively, at a pH from 10 to 12.5, and wherein molar ratio of the compound of formula (II) to 1,2-dichloroethane is from 1: 1 to 1:15.

In one embodiment, the process relates to the preparation of the compound of formula (IA), wherein R represents H. In such a case, the compound of formula (IA) is the salt of *N,N-*di(2-hydroxybenzyl)ethylenediamine-*N,N*'-diacetic acid (HBED).

In another embodiment, the process relates to the preparation of the compound of formula (IA), wherein R represents CH₃ group. In such a case, the compound of formula (IA) is the salt of *N,N*'-di(2-hydroxy-5-methylbenzyl)ethylenediamine-*N,N*'-diacetic acid (5Me-HBED).

In one of the embodiments, the process of the invention relates to the preparation of the salt of formula (IA), wherein M represents sodium cation, i.e. HBED and 5Me-HBED disodium salts.

The process of the invention relates also to the preparation of the salt of formula (IA), wherein M represents potassium cation, i.e. HBED and 5Me-HBED dipotassium salts.

Preferably, alkylation reaction is carried out at 30 to 70°C, especially at 40 to 60°C. It has been found that too high reaction temperature leads to the decomposition of the product. When temperature is too low, reaction proceeds slowly.

Preferably, unreacted 1,2-dichloroethane is removed from the reaction mixture by decantation of an organic layer and then extraction of an aqueous phase with an organic solvent, preferably with ethyl acetate or isobutyl acetate, to obtain the compound of the formula (IA) in the form of an aqueous solution or suspension.

Preferably, the compound of the formula (II) used in the process of the invention as described above is obtained by condensation of glycine with the compound of the formula (III) 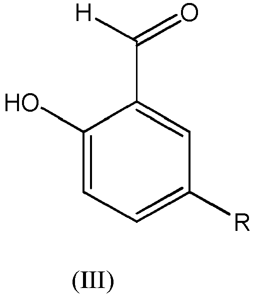 wherein R has the meaning as defined for the formula (IA), followed by reduction of the condensation product carried out without its isolation in the same reaction vessel, with hydrogen gas in the presence of a heterogeneous catalyst.

In the process of the invention condensation of glycine with the compound of the formula (III) and reduction of the condensation product can be carried out in a polar solvent.

In the process of the invention condensation of glycine with the compound of the formula (III) and reduction of the condensation product can be alternatively carried out in water, in the presence of sodium or potassium hydroxide. In such a case, reaction mixture after reduction of the condensation product and separation of the catalyst is neutralized with an acid.

In the process of the invention condensation of glycine with the compound of the formula (III) and reduction of the condensation product can be also carried out in water in the presence of a tertiary amine, preferably triethylamine, as a replacement of sodium or potassium ions. In such a case, reaction mixture after reduction of the condensation product and separation of the catalyst is alkalized to a pH at least 12, organic phase containing the tertiary amine is separated from aqueous phase, and aqueous phase is neutralized with an acid.

In the first of the variants, condensation of glycine with the compound of the formula (III) and reduction of the condensation product to obtain the compound of the formula (II) is carried out in a polar solvent. Most often methyl alcohol, isopropyl alcohol or methyl alcohol - water mixture are used. Molar ratio of the compound of the formula (III) to glycine is from 1:1 to 1:1.05. pH of the reaction mixture after completion of dosing of reagents is in the range from 9 to 10.5.

In the second variant, condensation of glycine with the compound of the formula (III) and reduction of the condensation product to obtain the compound of the formula (II) is carried out in water, in the presence of sodium or potassium hydroxide. Reaction mixture after reduction of the condensation product and separation of the catalyst, preferably by filtration, especially by pressure filtration, is neutralized with an acid. This variant of carrying out the reaction is advantageous, since in such a case a homogenous mixture is obtained. For this purpose, glycine is neutralized with sodium or potassium hydroxide in a molar ratio from 1:0.8 to 1:1 (glycine : sodium or potassium hydroxide), then the compound of the formula (III) is added dropwise (salicylaldehyde when R represents H or 2-hydroxy-5-methylbenzaldehyde when R represents CH₃ group). After reduction of the condensation product, soluble in water intermediate sodium or potassium *N*-(2-hydroxybenzylidene)glycinate (when R represents H) or soluble in water intermediate sodium or potassium *N*-(2-hydroxy-5-methylbenzylidene)glycinate (when R represents CH₃ group) is obtained. To obtain the compound of the formula (II), reaction mixture after reduction and separation of the catalyst is neutralized with an acid, preferably hydrochloric acid, such as 37% hydrochloric acid, to pH=4. Precipitated solid compound of the formula (II) is filtered and dried.

In the third variant, condensation of glycine with the compound of the formula (III) and reduction of the condensation product to obtain the compound of the formula (II) is carried out in water, in the presence of tertiary amine, for example triethylamine. In such a case, after reduction of the condensation product and separation of the catalyst, reaction mixture is alkalized to pH at least 12 to separate triethylamine containing organic phase and aqueous phase (in an alkaline environment amine isolates from aqueous solution). To recover the compound of the formula (II), separated aqueous phase is subsequently acidified to pH=4, preferably with hydrochloric acid, such as 37% hydrochloric acid. Thus precipitated solid compound of the formula (II) is filtered and dried.

Reduction of *N*-(2-hydroxybenzylidene)glycine and *N*-(2-hydroxy-5-methyl-benzylidene)glycine or their sodium or potassium salts to glycine derivative of the formula (II) is carried out directly in the reaction mixture obtained after condensation of aldehyde of the formula (III) and glycine. Reduction is carried out under hydrogen pressure of 1-10 bar and in the presence of a heterogeneous catalyst. Preferred catalyst is palladium or platinum supported on an inorganic carrier, carbon, alumina, or silica, especially palladium on carbon (Pd/C). In each of the presented variants, after completion of the reduction heterogenous catalyst is isolated from reaction mixture by filtration, preferably by pressure filtration. Recovered catalyst can be used in a further reduction reaction. After multiple use, the catalyst is regenerated and re-activated.

If condensation reaction is carried out in methanol, reduction takes place in a homogenous mixture when large amount of a solvent is used, or in a suspension when maximum solubility of the compound of formula (III) in an organic solvent is exceeded.

If water is used as a solvent and sodium or potassium hydroxide, reduction takes place in a completely homogenous mixture, since *N*-(2-hydroxybenzylidene)glycinates and *N*-(2-hydroxy-5-methylbenzylidene)glycinates are soluble in water.

Alkylation of glycine derivative of the formula (II) with 1,2-dichloroethane proceeds in a two-phase system. Reaction takes place in aqueous medium in the presence of sodium or potassium hydroxide, the second phase is alkylating agent, 1,2-dichloroethane. 1,2-Dichloroethane is used in molar excess with respect to glycine derivative of the formula (II). Molar ratio of glycine derivative of the formula (II) to 1,2-dichloroethane is in the range from 1: 1 to 1:15, preferably form 1:5 to 1:15, especially from 1:5 to 1:10. Alkylation is carried out at 30-70°C, preferably 40-60°C. After completion of the reaction, the unreacted alkylating agent is removed by decantation of upper layer of 1,2-dichloroethane (organic layer).

Additionally, the residue of 1,2-dichloroethane is removed from aqueous layer by extraction with an organic solvent. Solvents such as ethyl acetate or isobutyl acetate are used for extraction. Extraction operation is carried out after lowering pH of the aqueous layer to 8-10.

Extraction product (raffinate) thus obtained in the form of an aqueous solution or suspension of sodium or potassium salt of the formula (IA) is a starting material for synthesis of chelates of microelements.

Re-use of decanted 1,2-dichloroethane is possible by recirculation to further reaction cycles. After multiple use, 1,2-dichloroethane is regenerated by fractional distillation, wherein fraction boiling at 65-72°C is collected of purity above 90%. Starting material thus regenerated is ready for re-use in production.

Sodium and potassium salts of *N*,*N*'-disubstituted ethylenediamine-*N,N*'-diacetic acid derivatives represented by the formula (IA) and obtained using the process described above can be optionally converted into acid addition salts, in particular monohydrochloride and monosulfate.

The object of the invention is therefore also a process of the preparation of acid addition salts of *N,N*'-disubstituted ethylenediamine-*N,N*'-diacetic acid derivatives represented by the formula (IB) 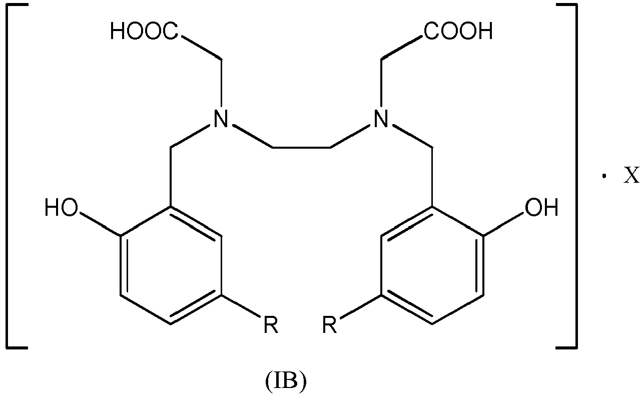 wherein both R have the same meaning and represent hydrogen atom or CH₃ group, and X represents HCl or H₂SO₄, i.e. monohydrochloride and monosulfate.

According to the invention, an aqueous solution of the compound of the formula (IA) obtained by the process as described above is acidified to pH in the range 0.5-2.0, with an acid selected from the group consisting of hydrochloric acid and sulfuric(VI) acid, respectively, and optionally precipitate of said addition salt is isolated.

Preferably, acidification is carried out without isolation of the compound of the formula (IA) from the aqueous solution or suspension obtained after removal of 1,2-dichloroethane from the reaction mixture after said alkylation with 1,2-dichloroethane.

The object of the invention is also a process for the preparation of iron(III) and zinc(II) chelates of N,N'-disubstituted ethylenediamine-N,N'-diacetic acid derivatives, which comprises preparation of the salts of N,N'-disubstituted ethylenediamine-N, N-diacetic acid derivatives of the formula (IA) according to the process of any one of claims 1 to 11, or acid addition salts of N,N' disubstituted ethylenediamine-N,N'-diacetic acid derivatives of the formula (IB) according to the process of any one of claims 12 to 13, and chelation reaction of the compounds of the formula (IA) or (IB) thus obtained with a salt of iron(II) or iron(III), or a salt of zinc(II), respectively.

If compound of the formula (IA) is used for chelation, chelation process is preferably carried out using aqueous solution or suspension of the sodium or potassium salt obtained directly after extraction of 1,2-dichloroethane phase (raffinate). If compound of the formula (IB) is used for chelation, chelating agent in the form of hydrochloride or sulfate is first dissolved in water with the addition of stoichiometric amount of sodium or potassium hydroxide, required for neutralization of chloride or sulfate ions and obtaining monosodium or monopotassium salt. Iron source are iron(II) or iron(III) salts, most often iron(II) nitrate(V), iron(II) chloride, iron(III) chloride, iron(II) sulfate(VI) and iron(III) sulfate(VI).

Preferably, chelation is carried out with aqueous solutions of iron salts mentioned above. Chelation takes place in an aqueous medium at pH=7-11.5 and at 20-50°C, preferably at 20-30°C. Chelation process begins with lowering of the pH of the aqueous solution or suspension to 7-9 with mineral acid (hydrochloric acid or sulfuric(VI) acid). At the same time, an aqueous solution of iron salt and additional amount of sodium or potassium hydroxide is added to maintain pH in the range 7 to 11. Preferably, chelation process is carried out at the pH in the range from 7 to 9.

Depending on the amount of the solvent (water) used for chelation, a solution or suspension of iron(III) chelate is obtained. In both cases obtained mixture is spray-dried to remove water and obtained product is additionally granulated. Alternatively, after completion of the chelation, suspension is filtered under pressure to lower the content of inorganic salts in the product. Obtained precipitate of iron(III) chelate in the form of sodium or potassium salt is dried at 105-125°C to dry mass. Iron(III) chelate having Fe(III) concentration 7-8% by weight is thus obtained. For removal of water from the chelate suspension conventional industrially available methods are used, consisting in evaporation of water at the temperature above 100°C or under reduced pressure. Methods of drying combined with mechanical granulation in rotary granulators or fluid-bed granulators are used.

Zinc source for the preparation of zinc(II) chelates with HBED and 5Me-HBED is zinc(II) carbonate(IV). Chelation of zinc is carried out in water in an acidic medium at pH in the range from 0.5 to 1.5. Molar ratio of the chelating agent to zinc ions is in the range from 1:1 to 1:0.95. Chelating agent is added in the form of monohydrochloride or monosulfate isolated from the reaction mixture after extraction of the excess of alkylating agent 1,2-dichloroethane. Aqueous suspension of the chelating agent and zinc(II) carbonate(IV) is stirred for 2 to 12 hours at 20-80°C, preferably at 25-35°C. Chelation process proceeds *in situ* to form zinc(II) chelate in an acidic, water-insoluble form and stoichiometric amount of carbon dioxide.

After cease of release of carbon dioxide from reaction mixture, which is characteristic for the completion of chelation process, zinc chelate in the acidic form is dissolved by the addition of stoichiometric amount of sodium or potassium hydroxide. Complete homogenization of the mixture and conversion of zinc(II) chelate into water-soluble form is reached at the pH in the range 8-12, preferably in the pH range 8-10. Concentration of Zn(II)HBED chelate is 15-20% w/w. Solid product is obtained by evaporation of water to its content in the final product of 0.5-5% w/w. Evaporation is carried out using conventional methods. Product is mechanically granulated to prevent dust formation in the commercial product.

### Examples

### Analytical methods

**HPLC for determining the content of chelating agents HBED and 5M-HBED:** according to EN 13368-2:2018-02 standard.

HPLC analyses were conducted using a Dionex UltiMate 3000 unit equipped with a UV-Vis-DAD detector, operating at 280 nm wavelength. Thermo Scientific Hypersil Gold C18 (150 mm/4.6 mm with stationary particle size of 5 µm) was used as a column. The mobile phase was a gradient mixture of 0.1 mol ammonium formate and acetonitrile at a ratio of 100:0 at 0 min, 40:60 at 11.6 min, 90:10 at 20 min, and 100:0 at 22 min, at a flow rate of 1 ml min⁻¹. The time of analysis was 20 minutes, and the injection volume was equal to 20 µl. Concentrations were determined on the basis of a calibration curve in the range of 10-500 ppm.
**HPLC for determining the content of zinc(II) and iron(III) chelated by HBED and**
**5Me-HHBED:** according to EN 13368-2:2018-02 standard.

### Preparation of starting materials

### Example 1: Preparation of N-(2-hydroxybenzyl)glycine (in methanol as a solvent)

Heated autoclave equipped with a mechanical stirrer was charged with 100 ml of methanol, and 7.58 g (100 mmol) of glycine (99% purity). To this solution, 12.59 g (100 mmol) of salicylaldehyde (97% purity) were added dropwise over a period of 5 minutes. The mixture was stirred for 60 minutes at room temperature until complete homogeneity was achieved. Then, the catalyst Pd/C was added in the amount of 1% by weight in respect to salicylaldehyde. Then, air was removed from the reaction system using the flow of nitrogen, and the autoclave was pressurized with hydrogen gas. The reduction process was carried out for 5 hours at a hydrogen pressure of 5 bar and a temperature of 65°C. Then, the hydrogen was removed from the reaction system using the flow of nitrogen, and the catalyst was filtered out. Then, the methanol was removed using a rotary evaporator, and the mixture was cooled to room temperature. Then, 50 ml of water were added, the precipitate was filtered out under reduced pressure, washed with 20 ml of water and dried under reduced pressure at 60°C. 15.57 g of *N*-(2-hydroxybenzyl)glycine was obtained with overall yield of 86%. The HPLC analysis showed a purity at a level of 96%. The structure was confirmed by means of ¹H NMR (D₂O) δ: 7.07-6.51 (m, 4H, ArH), 3.71 (s, 2H, ArCH₂N), 3.21 (s, 2H, NCH₂C), ¹³C NMR (D₂O) δ: 173.11, 157.55, 129.29, 128.44, 122.30, 121.08, 115.48, 51.31, 50.04; and the purity was confirmed by elemental analysis: C₉H₁₁NO₃, calculated: C 59.66%, H 6.12%, N 7.73%, O 26.49%, obtained: C 59.19%, H 6.41%, N 7.33%, O 26.99%.

### Example 2: Preparation of N-(2-hydroxybenzyl)glycine in aqueous solution in the presence of sodium hydroxide

Heated autoclave equipped with a mechanical stirrer was charged with 100 ml of water, 7.58 g (100 mmol) of glycine (99% purity), and 16.00 g of a 25% w/w solution (100 mmol) of sodium hydroxide. To this solution, 13.22 g (105 mmol) of salicylaldehyde (97% purity) were added dropwise over a period of 5 minutes. The mixture was stirred for 70 minutes at room temperature until complete homogeneity was achieved. Then, the Pd/C catalyst was added in the amount of 1% by weight in respect to salicylaldehyde. Then, air was removed from the reaction system using the flow of nitrogen, and the autoclave was pressurized with hydrogen gas. The reduction process was carried out for a period of 6 hours at a hydrogen pressure of 4 bar and a temperature of 70°C. Then, the hydrogen was removed from the reaction system using the flow of nitrogen, and the catalyst was filtered out. Then, the mixture was cooled to room temperature, acidified using 14.2 ml of 37% hydrochloric acid to pH=4, then cooled to room temperature again, and left without stirring overnight. Then, the precipitate formed was filtered out under reduced pressure, washed with 20 ml of water and dried under reduced pressure at 60°C. 16.47 g of *N*-(2-hydroxybenzyl)glycine were obtained in an overall yield of 91%. The HPLC analysis showed a purity at a level of 96%. The structure was confirmed by means of ¹H NMR (D₂O) δ: 7.02-6.54 (m, 4H, ArH), 3.72 (s, 2H, ArCH₂N), 3.24 (s, 2H, NCH₂C), ¹³C NMR (D₂O) δ: 173.11, 157.55, 129.29, 128.44, 122.30, 121.08, 115.48, 51.31, 50.04; and the purity was confirmed by elemental analysis: C₉H₁₁NO₃, calculated: C 59.66%, H 6.12%, N 7.73%, O 26.49%, obtained: C 59.22%, H 6.42%, N 7.12%, O 27.06%.

### Example 3: Preparation of N-(2-hydroxybenzyl)glycine in aqueous solution in the presence of triethylamine

Heated autoclave equipped with a mechanical stirrer was charged with 100 ml of water, 7.58 g (100 mmol) of glycine (99% purity) and 10.22 g (100 mmol) of triethylamine (99% purity). To this solution, 13.22 g (105 mmol) of salicylaldehyde (97% purity) were added dropwise over a period of 5 minutes. The mixture was stirred for 90 minutes at room temperature until complete homogeneity was achieved. Then, the Pd/C catalyst was added in the amount of 1% by weight in respect to salicylaldehyde. Then, air was removed from the reaction system using the flow of nitrogen, and the autoclave was pressurized with hydrogen gas. The reduction process was carried out for 4 hours at a hydrogen pressure of 6 bar and a temperature of 70°C. Then, the hydrogen was removed from the reaction system using the flow of nitrogen, and the catalyst was filtered out. Then, the mixture was cooled to room temperature and 48 g of a 25% w/w solution (300 mmol) of sodium hydroxide was added. After separation of phases, organic phase was collected, and aqueous phase was acidified using 36.30 ml of 37% hydrochloric acid to pH=4, cooled to room temperature again, and left without stirring overnight. Then, the precipitate was filtered out under reduced pressure, washed with 20 ml of water and dried under reduced pressure at 60°C. 14.84 g of *N*-(2-hydroxybenzyl)glycine were obtained with an overall yield of 82%. The HPLC analysis showed a purity at a level of 94%. The structure was confirmed by means of ¹H NMR (D₂O) δ: 7.00-6.50 (m, 4H, ArH), 3.70 (s, 2H, ArCH₂N), 3.20 (s, 2H, NCH₂C), ¹³C NMR (D₂O) δ: 173.11, 157.55, 129.29, 128.44, 122.30, 121.08, 115.48, 51.31, 50.04; and the purity was confirmed by elemental analysis: C₉H₁₁NO₃, calculated: C 59.66%, H 6.12%, N 7.73%, O 26.49%, obtained: C 59.15%, H 6.55%, N 7.13% O 26.50%.

### Example 4: Preparation of N-(2-hydroxy-5-methylbenzyl)glycine in aqueous solution in the presence of sodium hydroxide

Heated autoclave equipped with a mechanical stirrer was charged with 100 ml of water, 7.58 g (100 mmol) of glycine (99% purity), and 16.00 g of a 25% w/w (100 mmol) sodium hydroxide solution. To this solution, 14.74 g (105 mmol) of 2-hydroxy-5-methylbenzaldehyde (97% purity) were added dropwise over a period of 5 minutes. The mixture was stirred for 70 minutes at room temperature until complete homogeneity was achieved. Then, Pd/C catalyst was added in the amount of 1% by weight in respect to 2-hydroxy-5-methylbenzaldehyde. Then, air was removed from the reaction system using the flow of nitrogen, and the autoclave was pressurized with hydrogen gas. Reduction process was carried out for a period of 6 hours at a hydrogen pressure of 4 bar and a temperature of 70°C. Then, the hydrogen was removed from the reaction system using the flow of nitrogen, and the catalyst was filtered out. Then, the mixture was cooled to room temperature, acidified using 14.0 ml of 37% hydrochloric acid to pH=4, then cooled to room temperature again, and left without stirring overnight. Then, the precipitate formed was filtered out under reduced pressure, washed with 20 ml of water and dried under reduced pressure at 60°C. 16.47 g of N-(2-hydroxy-5-methylbenzyl)glycine were obtained in an overall yield of 92%. The HPLC analysis showed a purity at a level of 96%. The structure was confirmed by means of ¹H NMR (D₂O) δ: 7.12 - 6.65 (m, 3H, ArH), 3.72 (s, 2H, ArCH₂N), 3.24 (s, 2H, NCH₂C), 1.88 (s, 3H, ArCH₃) ¹³C NMR (D₂O) δ: 173.11, 157.55, 129.29, 128.44, 122.30, 121.08, 120.15, 115.48, 51.31, 50.04; and the purity was confirmed by elemental analysis: C₁₀H₁₃NO₃, calculated: C 61.53%, H 6.71%, N 7.18%, O 24.59%, obtained: C 60.44%, H 6.31%, N 6.99%, O 25.80%.

### Preparation of disodium and dipotassium salts

### Example 5: Preparation of the aqueous solution of N,N'-di(2-hydroxybenzyl)-ethylenediamine-N,N'-diacetic acid (HBED) disodium salt

A 100 ml flask with a magnetic stirrer and a reflux condenser was charged with 19.99 g (200 mmol) of 1,2-dichloroethane (99% purity) and heated to 60°C. A solution of 3.76 g (20 mmol) of *N*-(2-hydroxybenzyl)glycine (96% purity) and 3.20 g of 50% w/w (40 mmol) sodium hydroxide solution in 9 ml of water were added to the flask. The mixture was stirred at 55°C. After 3 hours, 1.60 g of 50% w/w solution (20 mmol) of sodium hydroxide were added to the mixture. After 3 hours, another 1.60 g of 50% w/w solution (20 mmol) of sodium hydroxide were added to the mixture. After 3 hours, another 1.60 g of 50% w/w (20 mmol) sodium hydroxide solution was added to the mixture. After final 3 hours, another 1.60 g of 50% w/w (20 mmol) sodium hydroxide solution were added to the mixture. The mixture was stirred at 55°C for another 8 hours. After 20 hours of total reaction time, the mixture was cooled to room temperature and left without stirring for 1 hour, which resulted in the separation of phases. The organic phase containing 95% of 1,2-dichloroethane was collected. The aqueous phase was washed 3 times with 20 ml of isobutyl acetate. After the extraction, 21.12 g of raffinate (an aqueous suspension of the title product) were obtained. The product assay in the raffinate was 15.11% by weight according to the HPLC analysis. The total yield of the reaction was 73.9%.

### Comparative Example 5a

The process was carried out as in Example 5, except that reaction temperature was 20°C instead of 60°C. Overall reaction yield was 12.1%, and the product assay in the raffinate was 2.18% by weight. Unreacted starting material assay in the raffinate was 14.9% by weight.

### Comparative Example 5b

The process was carried out as in Example 5, except that reaction temperature was 80°C instead of 60°C. After extraction, 19.85 g of the raffinate were obtained. No product was identified in the raffinate. HPLC analysis showed complex mixture of unidentified compounds, which may be degradation products.

### Comparative Example 5c

The process was carried out as in Example 5, except that stoichiometric ratio of *N*-(2-hydroxybenzyl)glycine to 1,2-dichloroethane (molar ratio 1:0.5) rather than excess of 1,2-dichloroethane was used, starting from 1.00 g (10 mmol) of 1,2-dichloroethane and 3.76 g (20 mmol) of *N*-(2-hydroxybenzyl)glycine. The overall reaction yield was 14.5% and the product assay in the raffinate was 2.78 by weight.

### Example 6: Preparation of an aqueous solution of N,N'-di(2-hydroxy-5-methylbenzyl)-ethylenediamine-N,N'-diacetic acid (5Me-HBED) disodium salt

A 100 ml flask with a magnetic stirrer and a reflux condenser was charged with 19.99 g (200 mmol) of 1,2-dichloroethane (99% purity) and heated to 60°C. A solution of 4.06 g (20 mmol) of *N*-(2-hydroxy-5-methylbenzyl)glycine (96% purity) and 3.20 g of 50% w/w (40 mmol) sodium hydroxide solution in 9 ml of water were added to the flask. The mixture was stirred at 55°C. After 3 hours, 1.60 g of 50% w/w (20 mmol) sodium hydroxide solution were added to the mixture. After 3 hours, another 1.60 g of 50% w/w (20 mmol) sodium hydroxide solution were added to the mixture. After another 3 hours, 1.60 g of 50% w/w (20 mmol) sodium hydroxide solution were added to the mixture. After the final 3 hours, another 1.60 g more of 50% w/w (20 mmol) sodium hydroxide solution were added to the mixture. The mixture was stirred at 55°C for another 8 hours. After 20 hours of total reaction time, the mixture was cooled to room temperature and left without stirring for 1 hour, which resulted in the separation of phases. The organic phase containing 95% of 1,2-dichloroethane was collected. The aqueous phase was washed 3 times with 20 ml of isobutyl acetate. After the extraction, 21.50 g of raffinate (aqueous solution of the title product) were obtained. The product assay in raffinate was 15.02% by weight according to the HPLC analysis. The overall yield of the reaction was 77.6%.

### Comparative Example 6a

The process was carried out as in Example 6, except that stoichiometric ratio of *N*-(2-hydroxy-5-methylbenzyl)glycine to 1,2-dichloroethane (molar ratio 1:0.5) rather than excess of 1,2-dichloroethane was used, starting from 1.00 g (10 mmol) of 1,2-dichloroethane and 4.06 g (20 mmol) of *N*-(2-hydroxy-5-methylbenzyl)glycine. The overall reaction yield was 15.2% and the product assay in the raffinate was 3.05 by weight.

### Example 7: Preparation of an aqueous solution of N,N'-di(2-hydroxybenzyl)-ethylenediamine-N,N'-diacetic acid (HBED) dipotassium salt

Proceeding analogously as in Example 5 and using suitable amounts of 50% w/w potassium hydroxide solution instead of sodium hydroxide, 22.05 g of raffinate (aqueous suspension of the title product) were obtained after extraction. HPLC analysis showed the product assay in the raffinate of 15.57% by weight. The overall reaction yield was 74.0%.

### Example 8: Preparation of an aqueous solution of N,N'-di(2-hydroxy-5-methylbenzyl)-ethylenediamine-N,N'-diacetic acid (5Me-HBED) dipotassium salt

Proceeding analogously as in Example 6 and using suitable amounts of 50% w/w of potassium hydroxide solution instead of sodium hydroxide, 22.30 g of raffinate (aqueous solution of the title product) were obtained after extraction. HPLC analysis showed the product assay in the raffinate of 16.55% by weight. The overall reaction yield was 75.0%.

### Preparation of acid addition salts

### Example 9: Preparation of solid N,N'-di(2-hydroxybenzyl)ethylenediamine-N,N'-diacetic acid (HBED) monohydrochloride

Aqueous solution (raffinate) of *N*,*N*'-di(2-hydroxybenzyl)ethylenediamine-*N*,*N*'-diacetic acid disodium salt obtained in Example 5 was acidified to pH=1.5 using 6.2 ml of 37% hydrochloric acid and cooled to room temperature. The mixture was left at room temperature without stirring overnight. Then, the precipitate formed was filtered under reduced pressure and washed with water. After drying under reduced pressure at 60°C, 3.66 g of *N,N*'-di(2-hydroxybenzyl)ethylenediamine-*N,N*'-diacetic acid monohydrochloride were obtained with an 84% yield. The HPLC analysis showed a purity at a level of 88%. The structure and the purity were confirmed by means of ¹H NMR (CDCl₃) δ: 7.20-6.65 (m, 8H, ArH), 3.85 (s, 4H, NCH₂CH₂), 2.72 (s, 4H, ArCH₂N), and elemental analysis: C₂₀H₂₄N₂O₆·HCl, calculated: C 61.84%, H 6.23%, N 7.21%, O 24.71%, obtained: C 59.21%, H 7.15%, N 6.59%, O 26.02%.

### Example 10: Preparation of solid N,N'-di(2-hydroxy-5-methylbenzyl)ethylenediamine-N,N'-diacetic acid (5Me-HBED) monohydrochloride

Aqueous solution (raffinate) of *N,N*'-di(2-hydroxy-5-methylbenzyl)ethylenediamine-*N,N*'-diacetic acid disodium salt obtained in Example 6 was acidified to pH=1.5 using 6.2 ml of 37% hydrochloric acid and cooled to room temperature. The mixture was left at room temperature without stirring overnight. Then, the precipitate formed was filtered under reduced pressure and washed with water. After drying under reduced pressure at 60°C, 4.37 g of *N,N*'-di(2-hydroxy-5-methylbenzyl)ethylenediamine-*N,N*'-diacetic acid monohydrochloride were obtained with an 82% yield. The HPLC analysis showed a purity at a level of 85%. The structure and the purity were confirmed by means of ¹H NMR (CDCl₃) δ: 7.25-6.60 (m, 8H, ArH), 3.84 (s, 4H, NCH₂CH₂), 2.78 (s, 4H, ArCH₂N), 1.85 (s, 3H, ArCH₃) and elemental analysis: C₂₂H₂₈N₂O₆·HCl, calculated: C 58.34%, H 6.45%, N 6.18%, O 21.19%, Cl 7.83%, obtained: C 58.99%, H 6.66%, N 6.50%, O 22.02%.

### Example 11: Preparation of solid N,N'-di(2-hydroxybenzyl)ethylenediamine-N,N'-diacetic acid (HBED) monosulfate

Aqueous solution (raffinate) of *N*,*N*'-di(2-hydroxybenzyl)ethylenediamine-*N*,*N*'-diacetic acid disodium salt obtained in Example 5 was acidified to pH=1.45 using 1.9 ml of 98% sulfuric acid and cooled to room temperature. The mixture was left at room temperature without stirring overnight. Then, the precipitate formed was filtered under reduced pressure and washed with water. After drying under reduced pressure at 60°C, 4.46 g of *N,N*'-di(2-hydroxybenzyl)ethylenediamine-*N*,*N*'-diacetic acid monosulfate were obtained with a 78% yield. The HPLC analysis showed a purity at a level of 85%. The structure and the purity were confirmed by means of ¹H NMR (CDCl₃) δ: 7.18-6.64 (m, 8H, ArH), 3.82 (s, 4H, NCH₂CH₂), 2.72 (s, 4H, ArCH₂N), and elemental analysis: C₂₀H₂₄N₂O₆·H₂SO₄, calculated: C 49.38%, H 5.39%, N 5.76%, O 32.89%, S 6.59%, obtained: C 49.01%, H 5.15%, N 5.49%, O 33.22%.

### Example 12: Preparation of solid N,N'-di(2-hydroxy-5-methylbenzyl)ethylenediamine-N,N'-diacetic acid (5Me-HBED) monosulfate

Aqueous solution (raffinate) of *N,N*'-di(2-hydroxy-5-methylbenzyl)ethylenediamine-*N,N*'-diacetic acid disodium salt obtained in Example 6 was acidified to pH=1.55 using 1.8 ml of 98% sulfuric acid and cooled to room temperature. The mixture was left at room temperature without stirring overnight. Then, the precipitate was filtered under reduced pressure and washed with water. After drying under reduced pressure at 60°C, 4.72 g of *N,N'-*di(2-hydroxy-5-methylbenzyl)ethylenediamine-*N,N*'-diacetic acid monosulfate were obtained with a 79% yield. The HPLC analysis showed a purity at a level of 86%. The structure and the purity were confirmed by means of ¹H NMR (CDCl₃) δ: 7.18-6.64 (m, 8H, ArH), 3.82 (s, 4H, NCH₂CH₂), 2.72 (s, 4H, ArCH₂N), 1.89 (s, 3H, ArCH₃) and elemental analysis: C₂₂H₂₈N₂O₆·H₂SO₄, calculated: C 51.35%, H 5.88%, N 5.44%, O 31.09%, S 6.23, obtained: C 50.88%, H 6.10%, N 5.20%, O 32.22%.

### Preparation of iron(III) chelates

### Example 13: Procedure for the preparation of solid iron(III) chelate of N,N'-di(2-hydroxybenzyl)ethylenediamine-N,N'-diacetic acid (Fe(III)HBED) sodium salt and solid iron(III) chelate of N,N'-di(2-hydroxy-5-methylbenzyl)ethylenediamine-N,N'-diacetic acid (Fe(III)5Me-HBED) sodium salt from the aqueous solution of chelating agent disodium salt

A round bottom flask equipped with a magnetic stirrer and a pH-meter was charged with a solution of the aqueous solution (raffinate) prepared in Example 5, containing 15.11 g (34.95 mmol) of HBED disodium salt, or 100 g of the aqueous solution (raffinate) prepared in Example 6, containing 16.29 g (35.42 mmol) of 5Me-HBED disodium salt, respectively. The mixture is acidified with 14.00 ml of 37% hydrochloric acid to pH=9. Then, the aqueous solution of an iron(II) or iron(III) salt, containing 1.88 g (33.66 mmol) of iron, selected from the following:
- iron(II) chloride (14.41 ml of the aqueous solution at 13.04% Fe w/V)
- iron(III) chloride (10.50 ml of the aqueous solution at 17.9% Fe w/V)
- iron(II) sulfate(VI) (10.62 ml of the aqueous solution at 17.7% Fe w/V)
- iron(III) sulfate(VI) (10.16 ml of the aqueous solution at 18.5% Fe w/V), is added.

During the addition of the aqueous solution of the iron salt, pH is maintained in a range of 6-9 with a 25% w/w sodium hydroxide solution. After the addition, the mixture is stirred for 3 hours at room temperature. If iron(II) salts are used, 4.59 g of 50% w/w hydrogen peroxide solution (67.30 mmol) is then added dropwise at this moment. After stirring for an additional 3 hours, the mixture is alkalized with a 25% w/w solution of sodium hydroxide to pH=11.5. The mixture is stirred for 3 hours at room temperature. After this time, the precipitate formed is filtered under reduced pressure, dried for 3 hours at 105°C, and assayed for an iron(III) content according to EN 13368-2:2018-02 standard.

In accordance with the above procedure:
A. HBED disodium salt, iron(II) chloride and 12.2 ml of 25% w/w sodium hydroxide solution for maintaining pH at 6-9 were used; 24.29 g of iron chelate Fe(III)HBED having iron(III) assay 7.35% by weight were obtained;
B. HBED disodium salt, iron(III) chloride and 10.4 ml of 25% w/w sodium hydroxide solution for maintaining pH at 6-9 were used; 25.28 g of iron chelate Fe(III)HBED having iron(III) assay 7.05% by weight were obtained;
C. HBED disodium salt, iron(II) sulfate(VI) and 7.5 ml of 25% w/w sodium hydroxide solution for maintaining pH at 6-9 were used; 24.77 g of iron chelate Fe(III)HBED having iron(III) assay 7.20% by weight were obtained;
D. HBED disodium salt, iron(III) sulfate(VI) and 8.0 ml of 25% w/w sodium hydroxide solution for maintaining pH at 6-9 were used; 24.21 g of iron chelate Fe(III)HBED having iron(III) assay 7.19% by weight were obtained;
E. 5Me-HBED disodium salt, iron(II) chloride and 11.9 ml of 25% w/w sodium hydroxide solution for maintaining pH at 6-9 were used; 24.55 g of iron chelate Fe(III)5Me-HBED having iron(III) assay 7.27% by weight were obtained;
F. 5Me-HBED disodium salt, iron(III) chloride and 10.4 ml of 25% w/w sodium hydroxide solution for maintaining pH at 6-9 were used; 23.99 g of iron chelate Fe(III)5Me-HBED having iron(III) assay 7.12% by weight were obtained;
G. 5Me-HBED disodium salt, iron(II) sulfate(VI) and 7.5 ml of 25% w/w sodium hydroxide solution for maintaining pH at 6-9 were used; 25.20 g of iron chelate Fe(III)5Me-HBED having iron(III) assay 7.01% by weight were obtained; and
H. 5Me-HBED disodium salt, iron(III) sulfate(VI) and 8 ml of 25% w/w sodium hydroxide solution for maintaining pH at 6-9 were used; 24.49 g of iron chelate Fe(III)5Me-HBED having iron(III) assay 7.23% by weight were obtained.

Fe(III)HBED and Fe(III)5Me-HBED chelates can be prepared in an analogous manner, using suitable amounts of dipotassium HBED salt or dipotassium 5Me-HBED instead of disodium salts, and suitable amounts of potassium hydroxide instead of sodium hydroxide.

### Example 14: Procedure for the preparation of solid iron(III) chelate of N,N'-di(2-hydroxybenzyl)ethylenediamine-N,N'-diacetic acid (Fe(III)HBED) sodium salt and solid iron(III) chelate of N,N'-di(2-hydroxy-5-methylbenzyl)ethylenediamine-N,N'-diacetic acid (Fe(III)5Me-HBED) sodium salt from monohydrochloride or monosulfate of chelating agent

To the aqueous suspension of monohydrochloride or monosulfate of the chelating agent prepared in Examples 9 to 12 aqueous solution of a base is added until pH=9 is reached, so as to obtain aqueous solution of the chelating agent at approx. 18% w/w. The base is selected from the group consisting of sodium hydroxide, potassium hydroxide, aqueous ammonia, and a mixture of sodium and potassium hydroxides. After homogenization, the aqueous solution of a selected iron salt is added, while pH in the range 6-9 is maintained during this addition using the same selected base. When the addition of the iron salt is completed, the mixture is stirred for 3 hour at room temperature. In case of using iron(II) salts, a hydrogen peroxide solution is added dropwise in this moment. After stirring for another 3 hours, the mixture is alkalized with a solution of the same selected base to a pH=11.5. The mixture is stirred for another 3 hours at room temperature. After this time, the mixture is dried for 3 hours at 105°C. The final product is assayed for iron(III) content in % by weight according to EN 13368-2:2018-02.

### Example 14A: Preparation of solid iron(III) chelate of N,N'-di(2-hydroxy-5-methylbenzyl)ethylenediamine-N,N'-diacetic acid (Fe(III)5Me-HBED) sodium salt from iron(II) chloride and 5Me-HBED monohydrochloride

A 250 ml round bottom flask equipped with a magnetic stirrer, reflux condenser, and pH-meter was charged with 82 g of water and 18 g (34.97 mmol) of *N,N*'-di(2-hydroxy-5-methylbenzyl)ethylenediamine-*N,N*'-diacetic acid (5Me-HBED) monohydrochloride (purity 88%). 6.12 ml of a 25% w/w sodium hydroxide solution were added to the slurry until a pH=9 was reached. After the complete homogenization, 14.23 ml (33.22 mmol) of 13.04% Fe w/V iron(II) chloride solution were added in portions. During the addition of iron(II) chloride, a pH in the range of 6-9 was maintained, using 11.0 ml of a 25% w/w sodium hydroxide solution. After the complete addition, the mixture was stirred for 3 hours at room temperature. Then, 4.51 g (66.44 mmol) of a 50% w/w hydrogen peroxide solution were added in portions, and the mixture was stirred for another 3 hours. Then, the mixture was alkalized to a pH=11.5 using 8.0 ml of a 25% w/w sodium hydroxide solution. After another 3 hours, the mixture was dried for 3 hours at 105°C. 30.41 g of iron(III) chelate of *N,N*'-di(2-hydroxy-5-methylbenzyl)ethylenediamine-*N*,*N*'-diacetic acid, Fe(III)5Me-HBED, were obtained. Iron(III) assay in the final product was 6.12%.

### Example 14B: Preparation of solid iron(III) chelate of N,N'-di(2-hydroxybenzyl)-ethylenediamine-N,N'-diacetic acid (Fe(III)HBED) from a solution of iron(II) chloride and HBED monohydrochloride

A 250 ml round bottom flask equipped with a magnetic stirrer, reflux condenser, and pH-meter was charged with 82 g of water and 18 g (37.28 mmol) of *N,N*'-di(2-hydroxybenzyl)ethylenediamine-*N,N*'-diacetic acid monohydrochloride (purity 88%). 6.20 ml of a 25% w/w sodium hydroxide solution were added to the slurry until a pH=9 was reached. After the complete homogenization, 15.17 ml (35.42 mmol) of a 13.04% Fe w/V iron(II) chloride solution were added in portions. During the addition of iron(II) chloride, a pH in the range of 6-9 was maintained, using 12.0 ml of a 25% w/w sodium hydroxide solution. After the complete addition, the mixture was stirred for 3 hours at room temperature. Then, 4.81 g (70.84 mmol) of a 50% w/w hydrogen peroxide solution were added in portions, and the mixture was stirred for another 3 hours. Then, the mixture was alkalized to a pH=11.5 using 8.0 ml of a 25% w/w sodium hydroxide solution. After another 3 hours, the mixture was dried at 105°C for 3 hours. 31.12 g of iron(III) chelate of *N*,*N*'-di(2-hydroxybenzyl)-ethylenediamine-*N*,*N*'-diacetic acid, Fe(III)HBED, were obtained. Iron(III) assay in the final product was 6.33%.

### Example 14C: Preparation of solid iron(III) chelate of N,N'-di(2-hydroxybenzyl)-ethylenediamine-N,N'-diacetic acid (Fe(III)HBED) from a solution of iron(III) chloride and HBED monohydrochloride

A 250 ml round bottom flask equipped with a magnetic stirrer, reflux condenser, and pH-meter was charged with 82 g of water and 18 g (37.28 mmol) of *N*,*N*'-di(2-hydroxybenzyl)ethylenediamine-*N,N*'-diacetic acid (HBED) monohydrochloride (purity 88%). 6.20 ml of a 25% w/w sodium hydroxide solution were added to the slurry until the pH reached 9. After complete homogenization, 11.03 ml (35.42 mmol) of 17.9% Fe w/V iron(III) chloride solution were added in portions. During the addition of iron(II) chloride, a pH in the range of 6-9 was maintained, using 10.5 ml of a 25% w/w sodium hydroxide solution. After complete addition, the mixture was stirred for 3 hours at room temperature. Then, the mixture was alkalized to a pH=11.5 using 8.0 ml of a 25% w/w sodium hydroxide solution. After another 3 hours, the mixture was dried for 3 hours at 105°C. 30.81 g of iron(III) chelate of *N*,*N*'-di(2-hydroxybenzyl)ethylenediamine-*N*,*N*'-diacetic acid, Fe(III)HBED, were obtained. Iron(III) assay in the final product was 6.28%.

### Example 14D: Preparation of solid iron(III) chelate of N,N'-di(2-hydroxy-5-methylbenzyl)ethylenediamine-N,N'-diacetic acid (Fe(III)5M-HBED) from a solution of iron(III) sulfate(VI) and 5Me-HBED monohydrochloride

A 250 ml round bottom flask equipped with a magnetic stirrer, reflux condenser, and pH-meter was charged with 82 g of water and 18 g (34.97 mmol) of *N,N*'-di(2-hydroxy-5-methylbenzyl)ethylenediamine-*N*,*N*'-diacetic acid (5Me-HBED) hydrochloride (purity 88%). 6.12 ml of a 25% w/w sodium hydroxide solution were added to the slurry until the pH reached 9. After complete homogenization, 10.28 ml (33.22 mmol) of a 18.5% Fe w/V solution of iron(III) sulfate were added in portions. During the addition of iron(III) sulfate, a pH in the range of 6-9 was maintained, using 8.0 ml of a 25% w/w sodium hydroxide solution. After complete addition, the mixture was stirred for 3 hours at room temperature. Then, the mixture was alkalized to a pH=11.5, using 8.0 ml of a 25% w/w sodium hydroxide solution. After another 3 hours, the mixture was dried for 3 hours at 105°C. 30.55 g of iron(III) chelate of *N,N*'-di(2-hydroxy-5-methylbenzyl)ethylenediamine-*N,N*'-diacetic acid, Fe(III)5Me-HBED, were obtained. Iron(III) assay in the final product was 6.00%.

Iron(III) chelates can be obtained analogously as in Examples 14 to 14D, using suitable amounts of HBED monosulfate or 5Me-HBED monosulfate.

### Preparation of zinc(II) chelates

### Example 15: Preparation of solid zinc(II) chelate of N,N'-di(2-hydroxybenzyl)-ethylenediamine-N,N'-diacetic acid (Zn(II)HBED) from zinc(II) carbonate(IV) solution and HBED monohydrochloride

A 250 ml round bottom flask equipped with a magnetic stirrer, reflux condenser, and pH-meter was charged with 82 g of water and 18 g (37.28 mmol) of *N*,*N*'-di(2-hydroxybenzyl)ethylenediamine-*N,N*'-diacetic acid (HBED) monohydrochloride (purity 88%). To this slurry, 4.49 g (35.42 mmol) of zinc(II) carbonate(IV) (99% purity) were added in portions. The mixture was stirred for 12 hours. Then, 6.80 ml of a 25% w/w sodium hydroxide solution were added to the slurry until the pH reached 9. After complete homogenization, the mixture was dried for 3 hours at 105°C. 33.02 g of zinc(II) chelate of *N*,*N*'-di(2-hydroxybenzyl)-ethylenediamine-*N*,*N*'-diacetic acid, Zn(II)HBED, were obtained. The final product zinc(II) assay was 7.02%.

### Example 16: Preparation of solid zinc(II) chelate of N,N'-di(2-hydroxy-5-methylbenzyl)ethylenediamine-N,N'-diacetic acid (Zn(II)5Me-HBED) from zinc(II) carbonate(IV) solution and 5Me-HBED monohydrochloride

A 250 ml round bottom flask equipped with a magnetic stirrer, reflux condenser, and pH-meter was charged with 82 g of water and 18 g (34.97 mmol) of *N,N*'-di(2-hydroxy-5-methylbenzyl)ethylenediamine-*N*,*N*'-diacetic acid (5Me-HBED) monohydrochloride (purity 88%). To this slurry, 4.21 g (33.22 mmol) of zinc(II) carbonate(IV) (99% purity) were added in portions. The mixture was stirred for 12 hours. 6.80 ml of a 25% w/w sodium hydroxide solution were added to the slurry until the pH reached 9. After complete homogenization, the mixture was dried for 3 hours at 105°C. 30.45 g of zinc(II) chelate of *N,N*'-di(2-hydroxy-5-methylbenzyl)ethylenediamine-*N,N*'-diacetic acid, Zn(II)5Me-HBED, were obtained. The final product zinc(II) assay was 6.80%.

## Claims

1. A process of the preparation of the salts of *N,N*'-disubstituted ethylenediamine-*N*,*N*'-diacetic acid derivatives, said salts being represented by the formula (IA) 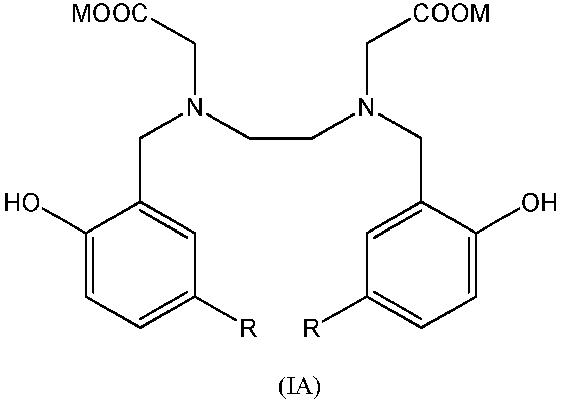
wherein both R have the same meaning and represent hydrogen atom or CH₃ group, and both M have the same meaning and represent sodium or potassium cation,
**characterized in that** it comprises alkylation reaction of A-substituted glycine derivative of the formula (II) 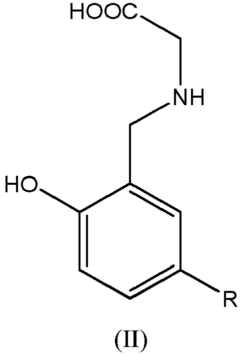
wherein R has the meaning as defined for the formula (IA),
with 1,2-dichloroethane in water in the presence of MOH hydroxide, wherein M represents sodium or potassium cation, respectively, at a pH from 10 to 12.5,
wherein molar ratio of the compound of the formula (II) to 1,2-dichloroethane is from 1:1 to 1:15.

2. The process of claim 1, wherein R represents hydrogen atom.

3. The process of claim 1, wherein R represents CH₃ group.

4. The process of anyone of claims 1 to 3, wherein M represents sodium cation.

5. The process of anyone of claims 1 to 3, wherein M represents potassium cation.

6. The process of anyone of claims 1 to 5, wherein alkylation reaction is carried out at 30 to 70°C.

7. The process of anyone of claims 1 to 6, wherein unreacted 1,2-dichloroethane is removed from the reaction mixture by decantation of an organic layer and then extraction of an aqueous phase with an organic solvent, preferably with ethyl acetate or isobutyl acetate, to obtain the compound of the formula (IA) in the form of an aqueous solution or suspension.

8. The process of anyone of claims 1 to 7, wherein the compound of the formula (II) is obtained by condensation reaction of glycine with the compound of the formula (III) 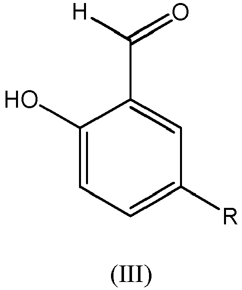 wherein R has the meaning as defined for the formula (I), followed by reduction of the condensation product without its isolation, in the same reaction vessel, with hydrogen gas in the presence of a heterogeneous catalyst.

9. The process of claim 8, wherein condensation and reduction are carried out in a polar solvent.

10. The process of claim 8, wherein condensation and reduction are carried out in water in the presence of sodium or potassium hydroxide, and reaction mixture after reduction of the condensation product and separation of the catalyst is neutralized with an acid.

11. The process of claim 8, wherein condensation and reduction are carried out in water in the presence of a tertiary amine, preferably triethylamine, and after reduction of the condensation product and separation of the catalyst reaction mixture is alkalized to a pH of at least 12 to separate organic phase containing the tertiary amine and aqueous phase, and aqueous phase is neutralized with an acid.

12. A process for the preparation of acid addition salts of *N,N*'-disubstituted ethylenediamine-*N*,*N*'-diacetic acid derivatives represented by the formula (IB) 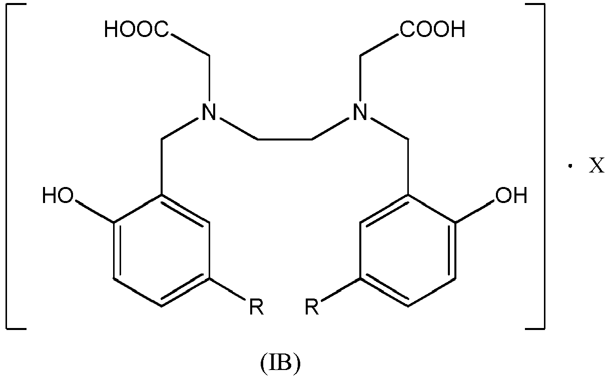 wherein both R have the same meaning and represent hydrogen atom or CH₃ group, and X represents HCl or H₂SO₄, **characterized in that** the salt of the formula (IA) is obtained in the process as defined in any one of claims 1 to 11, and then acidified in the aqueous solution to a pH in the range from 0.5 to 2.0 with an acid selected form the group consisting of HCl and H₂SO₄, respectively, and optionally precipitated solid addition salt is isolated.

13. The process of claim 12, **characterized in that** acidification is carried out without isolation of the compound of the formula (IA) from aqueous solution or suspension obtained after removal of unreacted 1,2-dichloroethane from reaction mixture after said alkylation with 1,2-dichloroethane.

14. A process for the preparation of iron(III) and zinc(II) chelates of *N*,*N*'-disubstituted ethylenediamine-*N*,*N*'-diacetic acid derivatives, which comprises preparation of the salts of *N,N*'-disubstituted ethylenediamine-*N*,*N*'-diacetic acid derivatives of the formula (IA) according to the process of any one of claims 1 to 11, or acid addition salts of *N,N'-*disubstituted ethylenediamine-*N*,*N*'-diacetic acid derivatives of the formula (IB) according to the process of any one of claims 12 to 13, and chelation reaction of the compounds of the formula (IA) or (IB) thus obtained with a salt of iron(II) or iron(III), or a salt of zinc(II), respectively.

## Patentansprüche

1. Verfahren zur Herstellung von Salzen von N,N'-disubstituierten Ethylendiamin-N,N'-diessigsäurederivaten, wobei die Salze durch die Formel (IA) 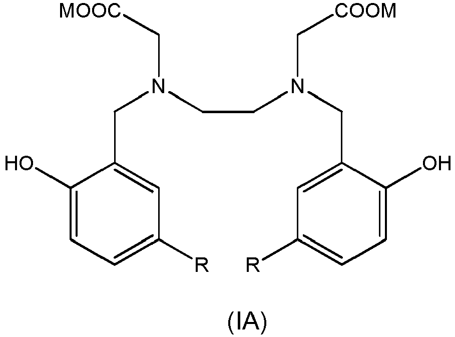 dargestellt werden, wobei beide R die gleiche Bedeutung haben und ein Wasserstoffatom oder eine CH₃-Gruppe darstellen, und beide M die gleiche Bedeutung haben und ein Natrium- oder Kaliumkation darstellen,
**dadurch gekennzeichnet, dass** es umfasst die Alkylierungsreaktion von N-substituiertem Glycinderivat der Formel (II) 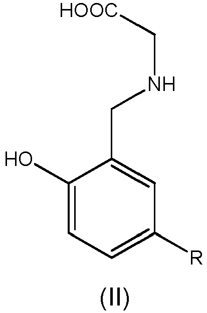 wobei R die für die Formel (IA) definierte Bedeutung hat,
mit 1,2-Dichlorethan im Wasser in Gegenwart von MOH-Hydroxid, wobei M Natriumbeziehungsweise Kaliumkation darstellt, bei einem pH-Wert von 10 bis 12,5,
wobei das Molverhältnis der Verbindung der Formel (II) zu 1,2-Dichlorethan 1:1 bis 1:15 wird.
wherein R has the meaning as defined for the formula (IA),

2. Verfahren nach Anspruch 1, wobei R ein Wasserstoffatom darstellt.

3. Verfahren nach Anspruch 1, wobei R eine CH₃-Gruppe darstellt.

4. Verfahren nach einem der Ansprüche 1 bis 3, wobei M Natriumkation darstellt.

5. Verfahren nach einem der Ansprüche 1 bis 3, wobei M Kaliumkation darstellt.

6. Verfahren nach einem der Ansprüche 1 bis 5, wobei die Alkylierungsreaktion bei 30 bis 70°C durchgeführt wird.

7. Verfahren nach einem der Ansprüche 1 bis 6, wobei nicht umgesetztes 1,2-Dichlorethan aus dem Reaktionsgemisch durch Dekantieren einer organischen Schicht und dann Extraktion einer wässrigen Phase mit einem organischen Lösungsmittel, vorzugsweise mit Ethylacetat oder Isobutylacetat, entfernt wird, um die Verbindung der Formel (IA) in Form einer wässrigen Lösung oder Suspension zu erhalten.

8. Verfahren nach einem der Ansprüche 1 bis 7, wobei die Verbindung der Formel (II) durch Kondensationsreaktion von Glycin mit der Verbindung der Formel (III) 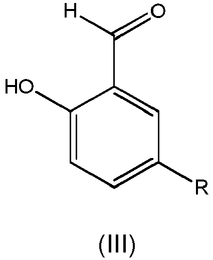 wobei R die für die Formel (I) definierte Bedeutung hat, gefolgt von Reduktion des Kondensationsprodukts ohne seine Isolierung, in demselben Reaktionsgefäß, mit Wasserstoffgas in Gegenwart eines heterogenen Katalysators erhalten wird.

9. Verfahren nach Anspruch 8, wobei Kondensation und Reduktion in einem polaren Lösungsmittel durchgeführt werden.

10. Verfahren nach Anspruch 8, wobei Kondensation und Reduktion in Wasser in Gegenwart von Natrium- oder Kaliumhydroxid durchgeführt werden und das Reaktionsgemisch nach Reduktion des Kondensationsprodukts und Abtrennung des Katalysators mit einer Säure neutralisiert wird.

11. Verfahren nach Anspruch 8, wobei Kondensation und Reduktion in Wasser in Gegenwart eines tertiären Amins, vorzugsweise Triethylamin, durchgeführt werden, und nach Reduktion des Kondensationsprodukts und Abtrennung des Katalysator, die Reaktionsgemisch auf einen pH von mindestens 12 alkalisiert wird, um die das tertiäre Amin enthaltende organische Phase und die wässrige Phase abzutrennen, und die wässrige Phase mit einer Säure neutralisiert wird.

12. Verfahren zur Herstellung von Säureadditionssalzen von N,N'-disubstituierten Ethylendiamin-N,N'-diessigsäurederivaten, die durch die Formel (IB) dargestellt werden 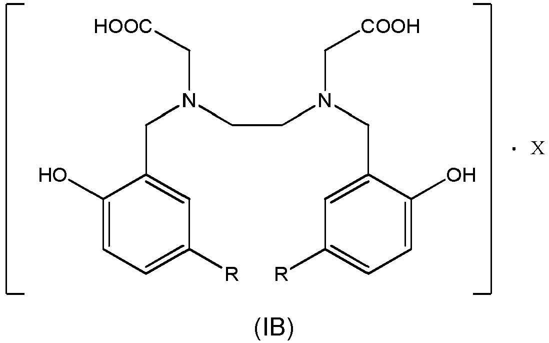 worin beide R die gleiche Bedeutung haben und ein Wasserstoffatom oder eine CH₃-Gruppe darstellen, und X HCl oder H₂SO₄ darstellt, **dadurch gekennzeichnet, dass** das Salz der Formel (IA) in dem Verfahren wie in einem der Ansprüche 1 bis 11 definiert erhalten wird und dann in der wässrigen Lösung mit einer Säure, ausgewählt aus der Gruppe, bestehend aus HCl bzw. H₂SO₄, auf einen pH im Bereich von 0,5 bis 2,0 angesäuert wird, und gegebenenfalls gefälltes festes Additionssalz isoliert wird.

13. Verfahren nach Anspruch 12, **dadurch gekennzeichnet, dass** das Ansäuern ohne Isolierung der Verbindung der Formel (IA) aus wässriger Lösung oder Suspension die nach Entfernung von nicht umgesetzten 1,2-Dichlorethan aus dem Reaktionsgemisch nach besagter Alkylierung mit 1,2-Dichlorethan erhaltend ist, durchgeführt wird.

14. Verfahren zur Herstellung von Eisen(III)- oder Zink(II)-chelaten von N,N'-disubstituierten Ethylendiamin-N,N'-diessigsäurederivaten, umfassend Herstellung von Salzen von N,N'-disubstituierten Ethylendiamin-N,N'-diessigsäurederivaten der Formel (IA) gemäß dem Verfahren nach einem der Ansprüche 1 bis 11, oder Herstellung von Säureadditionssalzen von N,N'-disubstituierten Ethylendiamin-N,N'-diessigsäure-derivaten der Formel (IB) gemäß dem Verfahren nach einem der Ansprüche 12 bis 13, und Chelatisierungsreaktion der so erhaltenen Verbindungen der Formel (IA) oder (IB) mit einem Salz von Eisen(II) oder Eisen(III) bzw. einem Salz von Zink(II).

## Revendications

1. Procédé de préparation des sels de dérivés d'acide éthylènediamine-N,N'-diacétique N,N'-disubstitués, lesdits sels étant représentés par la formule (IA) 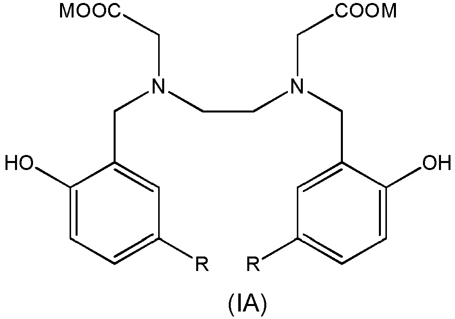 dans laquelle tous les deux R ont la même signification et représentent un atome d'hydrogène ou un groupe CH₃, et tous les deux M ont la même signification et représentent un cation sodium ou potassium,
**caractérisé en ce qu'** il comprend une réaction d'alkylation du dérivé de glycine N-substitué de formule (II) 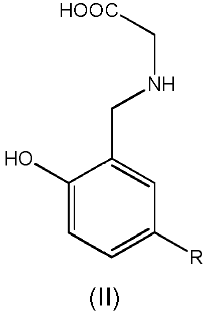 dans laquelle R a la signification telle que définie pour la formule (IA),
avec 1,2-dichloroéthane dans l'eau en présence d'hydroxyde MOH, où M représente un cation sodium ou potassium, respectivement, à un pH de 10 à 12,5,
dans lequel le rapport molaire du composé de formule (II) au 1,2-dichloroéthane est de 1:1 à 1:15.

2. Procédé selon la revendication 1, dans lequel R représente un atome d'hydrogène.

3. Procédé selon la revendication 1, dans lequel R représente un groupe CH₃.

4. Procédé selon l'une quelconque des revendications 1 à 3, dans lequel M représente un cation sodium.

5. Procédé selon l'une quelconque des revendications 1 à 3, dans lequel M représente un cation potassium.

6. Procédé selon l'une quelconque des revendications 1 à 5, dans lequel la réaction d'alkylation est effectuée à une température de 30 à 70 °C.

7. Procédé selon l'une quelconque des revendications 1 à 6, dans lequel le 1,2-dichloroéthane n'ayant pas réagi est éliminé du mélange réactionnel par décantation d'une couche organique et ensuite extraction d'une phase aqueuse avec un solvant organique, de préférence avec l'acétate d'éthyle ou l'acétate d'isobutyle, pour obtenir le composé de formule (IA) sous la forme d'une solution ou suspension aqueuse.

8. Procédé selon l'une quelconque des revendications 1 à 7, dans lequel le composé de formule (II) est obtenu par réaction de condensation de glycine avec le composé de formule (III) 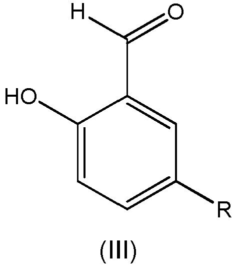 dans laquelle R a la signification telle que définie pour la formule (I), suivie par la réduction du produit de condensation sans son isolement, dans le même récipient de réaction, avec de l'hydrogène gazeux en présence d'un catalyseur hétérogène.

9. Procédé selon la revendication 8, dans lequel la condensation et la réduction sont effectuées dans un solvant polaire.

10. Procédé selon la revendication 8, dans lequel la condensation et la réduction sont effectuées dans l'eau en présence d'hydroxyde de sodium ou de potassium, et le mélange réactionnel après réduction du produit de condensation et séparation du catalyseur est neutralisé avec un acide.

11. Procédé selon la revendication 8, dans lequel la condensation et la réduction sont effectuées dans l'eau en présence d'une amine tertiaire, de préférence la triéthylamine, et après réduction du produit de condensation et séparation du catalyseur, le mélange réactionnel est alcalinisé à un pH d'au moins 12 pour séparer la phase organique contenant l'amine tertiaire et la phase aqueuse, et la phase aqueuse est neutralisée avec un acide.

12. Procédé de préparation de sels d'addition d'acide de dérivés d'acide éthylènediamine-N,N'-diacétique N,N'-disubstitués représentés par la formule (IB) 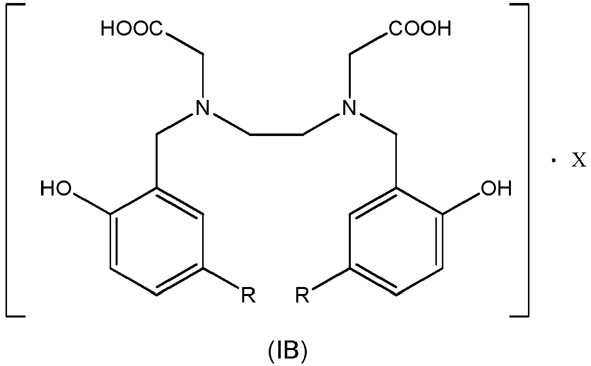 dans laquelle tous les deux R ont la même signification et représentent un atome d'hydrogène ou un groupe CH₃, et X représente HCl ou H₂SO₄, **caractérisé en ce que** le sel de formule (IA) est obtenu dans le procédé tel que défini dans l'une quelconque des revendications 1 à 11, et puis acidifié dans la solution aqueuse à un pH dans la plage de 0,5 à 2,0 avec un acide sélectionné dans le groupe constitué de HCl et H₂SO₄, respectivement, et éventuellement un sel d'addition solide précipité est isolé.

13. Procédé selon la revendication 12, **caractérisé en ce que** l'acidification est effectuée sans isolement du composé de formule (IA) d'une solution ou suspension aqueuse obtenue après élimination du 1,2-dichloroéthane n'ayant pas réagi du mélange réactionnel après ladite alkylation avec du 1,2-dichloroéthane.

14. Procédé de préparation des chélates de fer(III) et de zinc(II) des dérivés d'acide éthylènediamine-N,N'-diacétique N,N'-disubstitués, qui comprend préparation des sels de dérivés d'acide éthylènediamine-N,N'-diacétique N,N'-disubstitués de formule (IA) selon le procédé de l'une quelconque des revendications des revendications 1 à 11, ou des sels d'addition d'acide de dérivés d'acide éthylènediamine-N,N'-diacétique N,N'-disubstitués de formule (IB) selon le procédé de l'une quelconque des revendications 12 à 13, et réaction de chélation des composés de formule (IA) ou (IB) ainsi obtenus avec un sel de fer(II) ou de fer(III), ou un sel de zinc(II), respectivement.
